# EUROPÄISCHE PATENTSCHRIFT

(11) **EP 0 985 406 B1**
(45) Veröffentlichungstag und Bekanntmachung des Hinweises auf die Patenterteilung: **06.12.2000**
(21) Anmeldenummer: 99110869.7
(22) Anmeldetag: 07.06.1999
(51) Int. Cl.: A61K 7/13

(54) **Haarfärbemittel**
Hair dye composition
Composition pour la teinture des cheveux

(30) Priorität: 25.06.1998 DE 19828205
(43) Veröffentlichungstag der Anmeldung: 15.03.2000
(73) Patentinhaber: GOLDWELL GmbH, D-64297 Darmstadt (DE)
(72) Erfinder: Golinski, Frank Dr., 64297 Darmstadt (DE); Lorenz, Heribert, 64401 Gross-Bieberau (DE); Wagner, Helmar R., 64291 Darmstadt (DE)

(56) Entgegenhaltungen:
- EP-A- 0 079 540
- EP-A- 0 778 019
- DE-C- 19 807 245

## Beschreibung

Die vorliegende Erfindung betrifft ein Haarfärbemittel auf Basis eines mit Peroxid reagierenden Oxidationsfarbstoff-Systems, das dauerhafte intensive Farbtöne liefert, die entweder als solche angewandt werden, oder, in Kombination mit weiteren Entwickler- und/oder Kupplersubstanzen, zur Erzielung weiterer Farbnuancen benutzt werden können.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen ist insofern nicht völlig problemfrei, als sie bei extrem empfindlichen Personen in speziellen Fällen zu Hautsensibilisierung führen können (bei sogenannten "Para-Allergikern")

Es wurde bereits versucht, dieses Problem durch Verwendung alternativer Entwicklersubstanzen zu lösen. Dies ist in beschränktem Umfang möglich durch den Einsatz von Tetraaminopyrimidin; jedoch müssen dann Abstriche in der Farbintensität und den Variationsmöglichkeiten verschiedener Farbtöne hingenommen werden.

Eine weitere Lösung dieses Problems, nämlich die weitgehende Abwesenheit von Hautsensibilisierungen einerseits und eine größere Variationsbreite der Erzielung möglicher Farbnuancen andererseits, wird durch den in der EP-A 615 743 beschriebenen Einsatz von 2-(2'-Hydroxyethylamino)-5-aminotoluol bzw. dessen wasserlöslichen Salzen und der aus der EP-B 467 026 bekannten Triaminohydroxypyrimidine, insbesondere 2,5,6-Triamino-4-hydroxypyrimidin, 2,4,5-Triamino-6-hydroxypyrimidin, 4,5,6-Triamino-2-hydroxypyrimidin bzw. deren Salze, insbesondere die Sulfate, als Entwicklersubstanzen in Haarfärbemitteln erreicht.
Auch dann bleiben jedoch noch farbtechnische Wünsche offen.

Aus der DE-A 196 14 303 sind daher auch Oxidationshaarfärbemittel bekannt, die 2-(2'-Hydroxyethylamino)-5-aminotoluol bzw. dessen Salze als Entwicklersubstanzen in Kombination mit 2,6-Dichlor-4-aminophenol enthalten.

Obwohl durch diese Kombination eine Vielzahl von unterschiedlichen Farbtönen erzeugt werden kann, ist auch hier noch eine Optimierung sowohl der Variationsbreite als auch der Farbintensität möglich.

Es wurde nunmehr gefunden, daß eine intensive, licht-, wasch- und dauerwellstabile Haarfärbung, die auch keinerlei Hautirritationen und Hautsensibilisierung hervorruft dann erreicht werden kann, wenn man ein mit Peroxid reagierendes Oxidationsfarbstoff-System verwendet, das eine Kombination aus 2-(2,5-Diaminophenyl)ethanol bzw. dessen wasserlöslichen Salzen und 2,6-Dichlor-4-aminophenol enthält.

Bei Anwendung dieser Zusammensetzungen auf Basis einer üblichen Grundlage werden nach der Oxidation mit Peroxid sehr ausdrucksvolle, intensive, dauerhafte Haarfärbungen erhalten, die durch Zusatz entsprechender Kuppler- und weiterer Entwicklersubstanzen noch zu anderen Farbnuancen variiert werden können.

Solche bevorzugten Substanzen sind insbesondere ausgewählt aus der Gruppe 1-Methoxy-2-amino-4-(β-hydroxyethylamino)benzol, 2-Amino-3-hydroxypyridin, 2,6-Diaminopyridin, 3-Amino-2-methylamino-6-methoxypyridin, 2-(Dimethylamino)-5-aminopyridin, 1-(β-Hydroxyethyl)-2,5-diaminobenzol, 2-(2'-Hydroxyethylamino)-5-aminotoluol, 2,5,6-Triamino-4-hydroxypyrimidin, 5-Amino-2-methylphenol, 4-Amino-3-methylphenol, 2,5-Diaminopyridin, 2-Amino-5-N,N-diethylaminotoluol, 1,4-Diaminobenzol, 2,5-Diaminotoluol, 1,3-Diaminobenzol, 1-Methyl-2-hydroxy-4-aminobenzol, Resorcin, 2-Methylresorcin, 4-Chlorresorcin, 1-Naphthol, 2-Aminophenol, 5-Amino-2-methoxyphenol, 2-Amino-4-(β-hydroxyethylamino)anisol und/oder 3-Aminophenol bzw. deren wasserlösliche Salze.

Aus der EP-A 7537 und der EP-B 400 330 ist die Verwendung von 2-(2,5-Diaminophenyl)ethanol als Entwicklersubstanz in Haarfärbemitteln bereits bekannt; jedoch lassen sich mit den dort beschriebenen Systemen die mit den erfindungsgemäßen Kombinationen erhaltenen Effekte nicht erzielen.

Auch die zusätzliche Mitverwendung weiterer, an sich bekannter Entwicklersubstanzen ist möglich. Neben den bereits oben genannten sind hierbei insbesondere noch 4-Aminophenol, 5-Aminosalicylsäure und/oder 1,2,4-Triaminobenzol zu erwähnen.

Die Gesamtkonzentration der Entwicklersubstanzen liegt üblicherweise zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.

Das Gewichtsverhältnis von 2-(2,5-Diaminophenyl)ethanol zu 2,6-Dichlor-4-aminophenol liegt dabei zwischen etwa 1:8 bis 8:1, vorzugsweise etwa 1:5 bis 5:1, insbesondere 1:2 bis 2:1.

Die Kupplersubstanzen als Reaktionspartner der Entwicklersubstanz(en) liegen in den erfindungsgemäßen Haarfärbemitteln etwa im gleichen molaren Anteil wie die Entwicklersubstanzen vor, d.h., also in Mengen von 0,05 bis 5,0 %, vorzugsweise 0,1 bis 4 %, insbesondere 0,5 bis 3 Gew.-% der Gesamtzusammensetzung (ohne Oxidationsmittel), wobei sich die Angaben jeweils auf den Anteil an freier Base beziehen.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfmdungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind.

Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d.h. nach Vermischung mit Peroxid, kann sowohl im schwach sauren, d.h. einem Bereich von 5,5 bis 6,9, im neutralen als auch im alkalischen Bereich, d. h. zwischen pH 7,1 und 9,5 liegen.

Im folgenden werden verschiedene Ausführungsbeispiele zur Erläuterung der Erfindung gegeben.

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfüm | 0,4 |
| Ammoniak, 25%-ig | 8,5 |
| Ammoniumchlorid | 0,5 |
| Panthenol | 0,8 |
| Wasser | ad 100,0 |

Die erfindungsgemäßen Oxidationsfarbstoff-Kombinationen wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar durch Aufbringen einer 1:1-Mischung aus Farbstoff-Vorprodukt und 6%iger Wasserstoffperoxid-Lösung und zwanzigminütiger Einwirkung bei Zimmertemperatur, folgendem Auswaschen und Trocknen.

Es wurden die folgenden Färbungen erzielt:

### Beispiel 1:

| | |
|---|---|
| 0,28 (Gew.-%) | 2-(2,5-Diaminophenyl)ethanolsulfat (A 80) |
| 0,20 | 2,6-Dichlor-4-aminophenol |
| 0,25 | 3-Aminophenol |

### Färbung:

Kräftiges Dunkelviolett.

### Beispiel 2:

| | |
|---|---|
| 0,28 (Gew.-%) | A 80 |
| 0,20 | 2,6-Dichlor-4-aminophenol |
| 0,32 | α-Naphthol |

### Färbung:

Kräftiges, ausdrucksvolles Azurblau.

### Beispiel 3:

| | |
|---|---|
| 0,28 (Gew.-%) | A 80 |
| 0,20 | 2,6-Dichlor-4-aminophenol |
| 0,28 | 1-Methyl-2-hydroxy-4-aminobenzol |

### Färbung:

Kräftiges, glänzendes Violett.

### Beispiel 4:

| | |
|---|---|
| 0,28 (Gew.-%) | A 80 |
| 0,20 | 2,6-Dichlor-4-aminophenol |
| 0,28 | 2-Methylresorcin |

### Färbung:

Kräftiges, glänzendes Olivbraun.

### Beispiel 5:

| | |
|---|---|
| 0,28 (Gew.-%) | A 80 |
| 0,20 | 2,6-Dichlor-4-aminophenol |
| 0,25 | 2-Amino-3-hydroxypyridin |

### Färbung:

Glänzendes Rotbraun.

### Beispiel 6:

| | |
|---|---|
| 0,28 (Gew.-%) | A 80 |
| 0,20 | 2,6-Dichlor-4-aminophenol |
| 0,25 | Resorcin |

### Färbung:

Glänzendes Goldbraun.

### Beispiel 7:

| | |
|---|---|
| 0,28 (Gew.-%) | A 80 |
| 0,20 | 2,6-Dichlor-4-aminophenol |
| 0,63 | 1-Methoxy-2-amino-4-(β-hydroxyethylamino)benzolsulfat |

### Färbung:

Glänzendes Tiefblau.

## Patentansprüche

1. Haarfärbemittel auf Basis eines mit Peroxid reagierenden Oxidationsfarbstoff-Systems, enthaltend eine Kombination aus
a) 2-(2,5-Diaminophenyl)ethanol bzw. dessen wasserlöslichen Salzen, und
b) 2,6-Dichlor-4-aminophenol.

2. Haarfärbemittel nach Anspruch 1, enthaltend die Bestandteile a) und b) in einem Gewichtsverhältnis zwischen 1:5 und 5:1.

3. Haarfärbemittel nach einem der Ansprüche 1 oder 2, enthaltend als weitere Kupplersubstanz(en) mindestens eine Komponente, ausgewählt aus der Gruppe 1-Methoxy-2-amino-4-(β-hydroxyethylamino)benzol, 2-Amino-3-hydroxypyridin, 2,6-Diaminopyridin, 3-Amino-2-methylamino-6-methoxypyridin, 2-(Dimethylamino)-5-aminopyridin, 1-(β-Hydroxyethyl)-2,5-diaminobenzol, 2-(2'-Hydroxyethylamino)-5-aminotoluol, 2,5,6-Triamino-4-hydroxypyrimidin, 5-Amino-2-methylphenol, 4-Amino-3-methylphenol, 2,5-Diaminopyridin, 2-Amino-5-N,N-diethylaminotoluol, 1,4-Diaminobenzol, 2,5-Diaminotoluol, 1,3-Diaminobenzol, 1-Methyl-2-hydroxy-4-aminobenzol, Resorcin, 2-Methylresorcin, 4-Chlorresorcin, 1-Naphthol, 2-Aminophenol, 5-Amino-2-methoxyphenol, 2-Amino-4-(β-hydroxyethylamino)anisol und/oder 3-Aminophenol bzw. deren wasserlösliche Salze.

## Claims

1. Hair dyeing composition on the basis of an oxidation dyestuff system reacting with peroxide, comprising a combination of
a) 2-(2.5-diaminophenyl)ethanol or the water-soluble salts thereof, and
b) 2.6-dichloro-4-aminophenyl.

2. Hair dyeing composition according to claim 1, comprising the components a) and b) in a weight proportion between 1:5 and 5:1.

3. Hair dyeing composition according to claims 1 or 2, comprising as further coupling substance(s) at least one component selected from the group 1-methoxy-2-amino-4-(β-hydroxyethyl amino)benzene, 2-amino-3-hydroxypyridine, 2.6-diaminopyridine, 3-amino-2-methyl amino-6-methoxypyridine, 2-(dimethyl amino)-5-aminopyridine, 1-(β-hydroxyethyl)-2.5-diaminobenzene, 2(2'-hydroxyethyl amino)-5-aminotoluene, 2.5.6-triamino-4-hydroxypyrimidine, 5-amino-2-methyl phenol, 4-amino-3-methyl phenol, 2.5-diamino pyridine, 2-amino-5-N.N-diethyl aminotoluene, 1.4-diaminobenzene, 2.5-diaminotoluene, 1.3-diaminobenzene, 1-methyl-2-hydroxy-4-aminobenzene, resorcinol, 2-methyl resorcinol, 4-chlororesorcinol, 1-naphthol, 2-aminophenol, 3-aminophenol, 5-amino-2-methoxyphenol, and/or 2-amino-4-(β-hydroxyethyl amino)anisole or the water-soluble salts thereof.

## Revendications

1. Composition pour la teinture des cheveux comprenant un système des colorants d'oxydation quel réagit avec des peroxides, contenant une combinaison de
a) 2-(2,5-diaminophényl)éthanol ou ses sels soluble dans l'eau, et
b) 2.6-dichloro-4-aminophénol.

2. Composition pour la teinture des cheveux selon la revendication 1, comprenant les composants a) et b) dans une proportion pondérale entre 1:5 et 5:1.

3. Composition pour la teinture des cheveux selon les revendications 1 ou 2, comprenant comme agents coupleurs additionels au moins un composant sélectioné parmi le groupe de 1-méthoxy-2-amino-4-(β-hydroxyéthyl amino) benzène, 2-amino-3-hydroxypyridine, 2.6-diaminopyridine, 3-amino-2-méthyl amino-6-méthoxypyridine, 2-(diméthyl amino)-5-aminopyridine, 1-(β-hydroxyéthyl)-2.5-diaminobenzène, 2-(2'-hydroxyéthyl amino)-5-aminotoluène, 2.5.6-triamino-4-hydroxpyrimidine, 5-amino-2-méthyl phénol, 4-amino-3-méthyl phénol, 2.5-diamino pyridine, 2-amino-5-N.N-diéthyl aminotoluène, 1.4-diaminobenzène, 2.5-diaminotoluène, 1.3-diaminobenzène, 1-méthyl-2-hydroxy-4-aminobenzène, résorcinol, 2-méthyl résorcinol, 4-chlororésorcinol, 1-naphthol, 2-aminophénol, 3-aminophénol, 5-amino-2-méthoxyphénol et/ou 2-amino-4-(β-hydroxyéthyl amino)-anisole ou ses sels soluble dans l'eau.
